(19) **Europäisches Patentamt / European Patent Office / Office européen des brevets**

(11) **EP 4 661 763 B1**

(12) **EUROPEAN PATENT SPECIFICATION**

(45) Date of publication and mention of the grant of the patent:
**08.07.2026 Bulletin 2026/28**

(21) Application number: **24703729.4**

(22) Date of filing: **02.02.2024**

(51) International Patent Classification (IPC):
**A61B 8/00** *(2006.01)* **A61B 8/08** *(2006.01)*
**A61M 16/00** *(2006.01)*

(52) Cooperative Patent Classification (CPC):
**A61B 8/5223; A61M 16/024;** A61B 8/08

(86) International application number:
**PCT/EP2024/052626**

(87) International publication number:
**WO 2024/165441 (15.08.2024 Gazette 2024/33)**

(54) **SYSTEMS FOR DETECTION OF POSITIVE END-EXPIRATORY PRESSURE (PEEP) USING DIAPHRAGMATIC ULTRASOUND**

SYSTEME ZUR DETEKTION VON POSITIVEM ENDEXPIRATORISCHEM DRUCK (PEP) UNTER VERWENDUNG VON MEMBRANULTRASCHALL

SYSTÈMES DE DÉTECTION DE PRESSION EXPIRATOIRE POSITIVE (PEEP) EN UTILISANT L'ÉCHOGRAPHIE DIAPHRAGMATIQUE

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR

(30) Priority: **09.02.2023 US 202363444353 P**

(43) Date of publication of application:
**17.12.2025 Bulletin 2025/51**

(73) Proprietor: **Koninklijke Philips N.V.**
**5656 AE Eindhoven (NL)**

(72) Inventors:
• **HAARTSEN, Jaap Roger**
**5656 AG Eindhoven (NL)**
• **BUIZZA, Roberto**
**5656 AG Eindhoven (NL)**
• **HETE, Bernard**
**5656 AG Eindhoven (NL)**
• **HENDRIKS, Cornelis Petrus**
**5656 AG Eindhoven (NL)**
• **WIEMKER, Rafael**
**5656AG Eindhoven (NL)**

• **KOEHLER, Thomas**
**5656AG Eindhoven (NL)**
• **SABCZYNSKI, Joerg**
**5656AG Eindhoven (NL)**
• **POLKEY, Michael**
**5656AG Eindhoven (NL)**

(74) Representative: **Philips Intellectual Property & Standards**
**High Tech Campus 34**
**5656 AE Eindhoven (NL)**

(56) References cited:
**US-A1- 2021 016 035     US-A1- 2022 401 674**

• **EMANUELE BERNARDI: "A New Ultrasound Method for Estimating Dynamic Intrinsic Positive Airway Pressure: A Prospective Clinical Trial", AMERICAN JOURNAL OF RESPIRATORY AND CRITICAL CARE MEDICINE, vol. 198, no. 3, 1 August 2018 (2018-08-01), US, pages 392 - 396, XP093154583, ISSN: 1073-449X, DOI: 10.1164/ rccm.201706-1292LE**

**Description**

The following relates generally to the respiratory therapy arts, mechanical ventilation arts, ventilator induced lung injury (VILI) prevention arts, and related arts.

<u>BACKGROUND</u>

Intrinsic positive end-expiratory pressure (PEEP), also known as autoPEEP or iPEEP, occurs when the expiratory time is shorter than the time needed to fully deflate the lungs to its functional residual capacity (FRC), preventing the lung and chest wall from reaching an elastic equilibrium point. This is sometimes referred to as 'gas trapping.' When this problem occurs, a portion of each subsequent tidal volume may be retained in the patient's lungs, a phenomenon sometimes referred to as breath stacking or dynamic hyperinflation, resulting in increased end-expiratory pressure in the alveoli. If this goes unrecognized, depending on the ventilation mode, it may result in barotrauma, volutrauma, tidal volume reduction, hypotension, patient-ventilator asynchrony, or death. The main pathophysiological factors involved are insufficient time for emptying the lung and/or expiratory flow limitation (EFL), e.g., due to dynamic small airway collapse due to pressure from hyperinflated lung units, mainly for chronic obstructive pulmonary disease (COPD).

Diaphragmatic ultrasonography (US) allows for quantification of diaphragm thickness, strain (rate) and excursion, and with this also the respiratory rate and duration of each contraction. Diaphragm thickness (expressed as thickening fraction) and strain reflect contractile activity and correlate well with diaphragmatic electrical activity and trans diaphragmatic pressure. Consequently, thickness and strain may be used as a surrogate for respiratory effort. Applications of diaphragmatic ultrasound include assessment of diaphragm function, atrophy detection, weaning prediction, and mechanical ventilation (MV) setting management.

Ultrasound (US) measurements of diaphragm thickness are typically quantified in terms of a diaphragm thickening fraction (TFdi or TFDI). Diaphragm thickening fraction measurements are carried out by an operator who looks at the patient and takes an ultrasound image at end inhalation and end exhalation. With suitable positioning of the ultrasound probe, typically in an intercostal position, the superior and inferior boundaries or surfaces of the diaphragm appear as contrast lines in the ultrasound image, and the diaphragm thickness is then the separation of these two contrast lines in the image (possibly with suitable correction of angular position of the ultrasound beam respective to the surface normal of the diaphragm plane). The diaphragm thickness fraction is then determined by subtracting the diaphragm thickness $T_{ei}$ measured at end inhalation from the diaphragm thickness $T_{ee}$ measured at end exhalation, and dividing the difference by the diaphragm thickness $T_{ee}$ at end-exhalation according to Equation 1:

$$TFdi = \frac{T_{ei} - T_{ee}}{T_{ee}} * 100\%  \qquad (1)$$

with *TFdi* being the diaphragm thickness fraction.

Presence of intrinsic PEEP is detected by automatic or manual inspection of flow curves (i.e., by checking whether the expiratory flow is still occurring just before start of a new inhalation). The value of the intrinsic PEEP could be obtained from an expiratory hold maneuver (i.e. by forcing the expiratory flow to zero), leading to an equilibration of alveoli and airway pressure, and measuring the airway pressure (equal to intrinsic PEEP). This works well for a passive patient during controlled mechanical ventilation.

However, for an actively breathing patient during spontaneous or assisted ventilation the presence of intrinsic PEEP causes the inspiratory effort to start during the expiration phase to overcome the intrinsic PEEP before triggering a new breath. Consequently, it is difficult to manually or automatically detect it by inspection of pressure and flow curves. Also, a reliable measurement of the intrinsic PEEP value is much more difficult in the presence of respiratory muscle activity since during an expiratory hold maneuver it is not possible to determine which amount of measured positive airway occlusion pressure is due to expiratory muscle activity. In this case one feasible method is to measure the drop in esophageal pressure (as a surrogate for the pleural pressure) just before start of inspiration, and subsequently subtract the part due to respiratory muscle activity determined from esophageal pressure. However, this method is not routinely used because of its invasive nature, the need for a dedicated ventilator system supporting esophageal pressure measurements and the need for time-consuming calibration steps.

An ultrasound method for estimating dynamic intrinsic positive airway pressure is described in Emanuele Bernardi, American Journal of Respiratory and Critical Care Medicine, vol. 198, no. 3, 1 August 2018, pages 392-396, US ISSN: 1073-449X, DOI: 10.1164/rccm.201706-1292LE.

The following discloses certain improvements to overcome these problems and others.

## SUMMARY

The invention is set out in the independent claims. Particular embodiments of the invention are set out in the dependent claims. Any subject matter contained herein that does not fall within the scope of the appended claims is considered as being useful for understanding the invention.

One advantage resides in noninvasively determining the intrinsic PEEP value for a mechanically ventilated but actively breathing patient.

Another advantage resides in adjusting mechanical ventilator operation to compensate for a non-zero intrinsic PEEP.

Another advantage resides in using a combination of ultrasound imaging data and at least one ventilator waveform to determine an intrinsic PEEP value for a mechanical ventilated patient.

A given embodiment may provide none, one, two, more, or all of the foregoing advantages, and/or may provide other advantages as will become apparent to one of ordinary skill in the art upon reading and understanding the present disclosure.

## BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.

FIGURE 1 diagrammatically shows an illustrative respiration monitoring device in accordance with the present disclosure.

FIGURE 2 shows an example flow chart of operations suitably performed by the system of FIGURE 1.

FIGURE 3 shows an example in which the onset of inhalation effort by the patient occurs before the mechanical ventilator begins to apply inhalation pressure.

FIGURE 4 shows an example flow chart of detection of an intrinsic PEEP value.

## DETAILED DESCRIPTION

As used herein, the singular form of "a," "an," and "the" include plural references unless the context clearly dictates otherwise. As used herein, statements that two or more parts or components are "coupled," "connected," or "engaged" shall mean that the parts are joined, operate, or co-act together either directly or indirectly, i.e., through one or more intermediate parts or components, so long as a link occurs. Directional phrases used herein, such as, for example and without limitation, top, bottom, left, right, upper, lower, front, back, and derivatives thereof, relate to the orientation of the elements shown in the drawings and are not limiting upon the scope of the claimed invention unless expressly recited therein. The word "comprising" or "including" does not exclude the presence of elements or steps other than those described herein and/or listed in a claim. In a device comprised of several means, several of these means may be embodied by one and the same item of hardware.

The following discloses using diaphragmatic ultrasound to detect and measure an intrinsic PEEP (iPEEP) value in an actively breathing patient undergoing mechanical ventilation therapy. Diaphragmatic ultrasound is a non-invasive imaging modality that is readily available in the ICU which is used for visualization of the diaphragm. Diaphragmatic ultrasound allows a clinician to determine the thickness and thickness fraction of the diaphragm from which respiratory activity and hence the onset of inspiration can be derived to determine the iPEEP value.

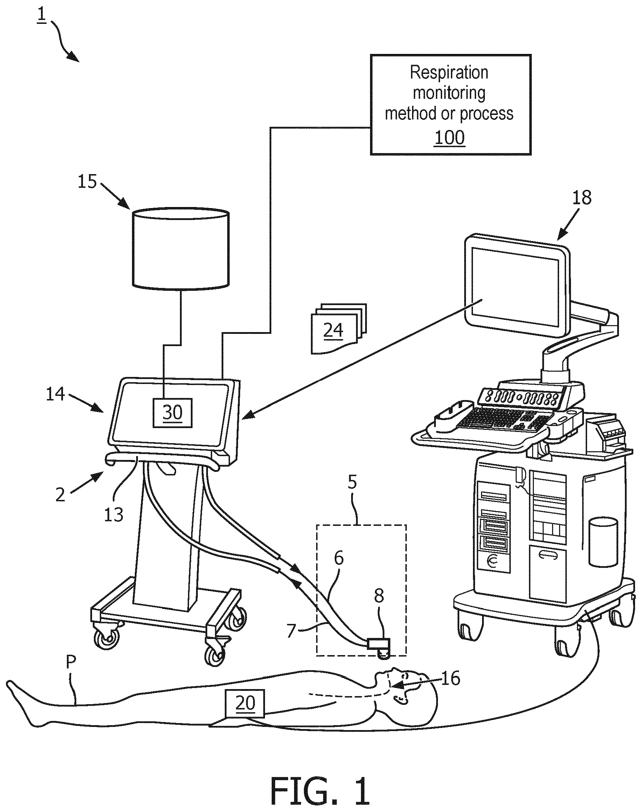

With reference to FIGURE 1, a diaphragm measurement device **1** is shown. A mechanical ventilator **2** is configured to provide ventilation therapy to an associated patient **P** is shown. As shown in FIGURE 1, the mechanical ventilator **2** is connected with a patient breathing circuit **5** to deliver mechanical ventilation to the patient **P.** The patient breathing circuit **5** includes typical components for a mechanical ventilator, such as an inlet line **6** (also called inhalation limb **6),** an optional outlet line **7** (also called exhalation limb **7;** this may be omitted if the ventilator employs a single-limb patient circuit), a connector or port **8** for connecting with an endotracheal tube (ETT) **16,** or a mask or other patient interface, and one or more breathing sensors (not shown), such as a gas flow meter, a pressure sensor, end-tidal carbon dioxide (etCO$_2$) sensor, and/or so forth. The mechanical ventilator **2** is designed to deliver air, an air-oxygen mixture, or other breathable gas (supply not shown) to the inhalation limb **6** of the patient breathing circuit **5** at a programmed pressure and/or flow rate to ventilate the patient via an ETT, and optionally also handling exhaled air received at the mechanical ventilator **2** via the exhalation limb 7 of the patient breathing circuit **5.** The mechanical ventilator **2** also includes at least one electronic processor or controller **13** (e.g., an electronic processor or a microprocessor), a display device **14,** and a non-transitory computer readable medium **15** storing instructions executable by the electronic controller **13.**

FIGURE 1 diagrammatically illustrates the patient **P** intubated with an ETT **16** (most of which is inside the patient

EP 4 661 763 B1

P and hence is shown in phantom). The connector or port **8** connects with the ETT **16** to operatively connect the mechanical ventilator **2** to deliver breathable air to the patient **P** via the ETT **16**. The mechanical ventilation provided by the mechanical ventilator **2** via the ETT **16** may be therapeutic for a wide range of conditions, such as various types of pulmonary conditions like emphysema or pneumonia, viral or bacterial infections impacting respiration such as a COVID-19 infection or severe influenza, cardiovascular conditions in which the patient **P** receives breathable gas enriched with oxygen, or so forth.

FIGURE 1 also shows a medical imaging device **18** (also referred to as an image acquisition device, imaging device, and so forth). As primarily described herein, the medical imaging device **18** comprises an ultrasound (US) medical imaging device **18.** The illustrative embodiments employ brightness mode (B-mode) ultrasound imaging to assess the diaphragm thickness metric. However, other types of ultrasound imaging or data are contemplated, such as motion mode (M-mode) data collected as a single ultrasound line over a time interval, A-mode, or so forth.

In some examples, the medical imaging device **18** includes an ultrasound probe **20** configured to acquire US imaging data (i.e., US images) **24** of the diaphragm of the patient **P**. In a more particular example, the medical imaging device **18** includes an ultrasound patch **20** that is wearable by the patient **P** (e.g., on the abdomen or chest of the patient **P** in position to image the diaphragm of the patient, as shown in FIGURE 1). The US patch **20** is positioned to acquire the US images **24** of the diaphragm of the patient **P**. For example, the US patch **20** is configured to acquire imaging data of a diaphragm of the patient **P,** and more particularly US imaging data related to a thickness of the diaphragm of a patient **P** during inspiration and expiration while the patient **P** undergoes mechanical ventilation therapy with the mechanical ventilator **2**. The electronic processor **13** controls the ultrasound imaging device **18** to receive the ultrasound imaging data **24** of the diaphragm of the patient **P** from the US patch **20**. Although only one US patch **20** is shown in FIGURE 1, it will be appreciated that any suitable number of patches can be attached to the patient **P.** The ultrasound patch(es) **20** allow for continuous and automatic determination of the diaphragm thickness data (Tdi) from the acquired ultrasound imaging data **24.**

The non-transitory computer readable medium **15** stores instructions executable by the electronic controller **13** to perform a respiration monitoring method or process **100**.

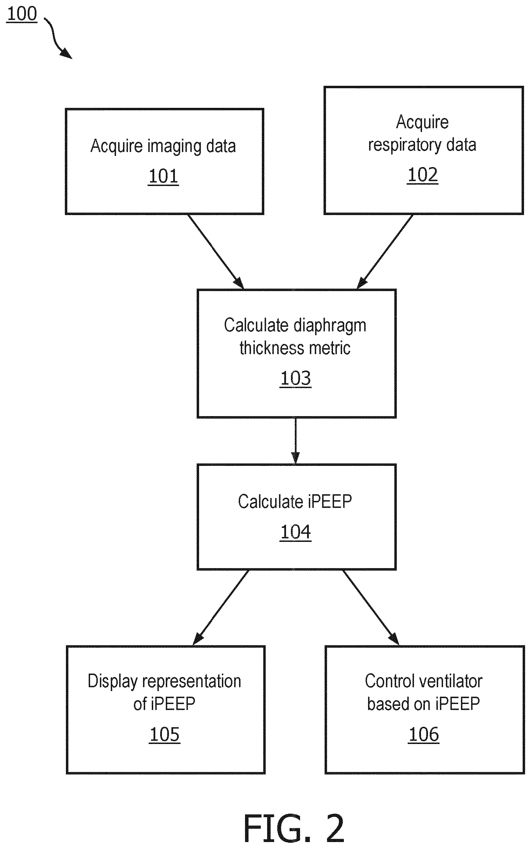

With reference to FIGURE 2, and with continuing reference to FIGURE 1, an illustrative embodiment of the respiration monitoring method **100** is diagrammatically shown as a flowchart. At an operation **101,** the US imaging data **24** of the diaphragm of the patient **P** is received as a function of time during inspiration and expiration while the patient undergoes mechanical ventilation therapy with the mechanical ventilator **2**. To do so, the electronic controller **13** can control the ultrasound patch **20** to acquire the ultrasound imaging data **24** and receive the ultrasound imaging data **24** of the diaphragm of the patient **P** from the ultrasound patch **20** and/or the medical imaging device **18 .**

At an operation **102,** respiratory data of the patient **P** during inspiration and expiration is received as a function of time while the patient undergoes the mechanical ventilation therapy with the mechanical ventilator **2**. Typically, the operations **101** and **102** are performed concurrently, that is, over the same time interval. The respiratory data of the patient **P** can include one or more of an airway pressure in an airway of the patient **P** or an airway flow in an airway of the patient **P** measured during ventilation therapy by the mechanical ventilator **2**. Notably, airway pressure and airway flow are commonly monitored during mechanical ventilation, so this data is typically available for any mechanically ventilated patient.

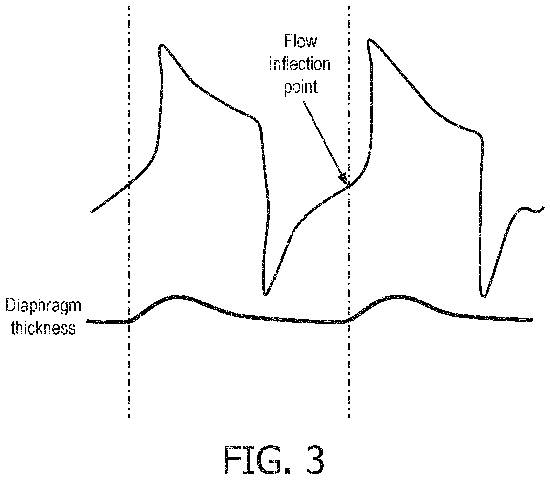

At an operation **103,** a diaphragm thickness metric as a function of time is calculated based on the received US imaging data **24** of the diaphragm of the patient **P.** As previously noted, with suitable positioning of the ultrasound probe in the operation **101,** typically in an intercostal position, the superior and inferior boundaries or surfaces of the diaphragm appear as contrast lines in the ultrasound image, and the diaphragm thickness is then measured as the separation of these two contrast lines in the image (possibly with suitable correction of angular position of the ultrasound beam respective to the surface normal of the diaphragm plane). However, unlike the case for determining the diaphragm thickening fraction or ratio (see Equation 1), for the iPEEP measurements disclosed herein the operation **103** determines diaphragm thickness as a function of time, with sufficient temporal resolution to detect onset of the inspiratory effort by the patient **P** as an onset of increased diaphragm thickness. This is seen in the lower curve of FIGURE 3, which shows the onset of inspiratory effort (marked as a dashed vertical line in FIGURE 3). The onset of inspiratory effort corresponds to a time of onset of an increase in the diaphragm thickness as the patient's inspiratory effort is executed by contraction of the diaphragm and its resultant thickening.

At an operation **104,** a iPEEP value for the patient **P** is calculated based on the ultrasound imaging data **24** and the respiratory data. For example, the iPEEP value is calculated based on the diaphragm thickness metric (from the diaphragm thickness metric calculation operation **103)** as a function of time and the respiratory data. To do so, a time corresponding to start of inhalation is identified based on (i) the determined diaphragm thickness metric, (ii) the received respiratory data, or (iii) a combination of both. In one example, the iPEEP value is calculated as $iPEEP = -R\dot{V}(t)$ where R is a respiratory resistance of lungs of the patient and V(t) is an airflow at the identified time corresponding to start of inhalation which is part of the received respiratory data of the patient. In another example, an updated iPEEP value applied by the mechanical ventilator **2** can be determined from the received respiratory data **24,** and a total PEEP value can be calculated as a sum of the calculated iPEEP value and the determined updated iPEEP value.

In some embodiments, the received respiratory data of the patient **P** as a function of time includes a ventilator flow waveform generated during the mechanical ventilation therapy. In this embodiment, the iPEEP calculation operation **104** include (i) detecting an onset of inspiratory effort by the patient **P** based on the ultrasound imaging data **24,** (ii) determining an air flow value from the lungs of the patient **P** at the detected onset of inspiratory effort by the patient **P** from the ventilator flow waveform, and (iii) calculating the iPEEP value based on the determined air flow value from the lungs of the patient **P** at the detected onset of inspiratory effort by the patient **P.** In some embodiments, the mechanical ventilator **2** is triggered to initiate a breath based on the determined onset of inspiratory effort by the patient **P.**

In a particular example, referring now to FIGURE 3, the onset of inspiratory effort is detected from ultrasound based diaphragmatic thickness measurements (i.e., at the start of the diaphragm thickening). Next, a corresponding air flow value is determined from a ventilator flow waveform shown in FIGURE 3. If the air flow is non-zero at this point, this is a sign of presence of iPEEP. Another indication of the presence of iPEEP is the time delay between the flow inflection point and the onset of the patient's inspiratory effort. This is because it takes time for the diaphragm (and other respiratory muscles) to first counterbalance the iPEEP for this effort to generate a small pressure drop (i.e., in the presence of a closed circuit) or to initiate the inspiratory flow (i.e., in an open circuit). The mechanical ventilator **2** and the ultrasound imaging device **18** need to be synchronized for accurate results, for example the respiratory muscle onset detected from the diaphragmatic ultrasound can be used to trigger the next breath when the ventilator and ultrasound machine are synchronized (see, e.g., U.S. Provisional App. No. 63/426,069, filed November 17, 2022).

FIGURE 3 shows an example of a ventilator flow waveform, which can be displayed on the display device **14.** A flow waveform (shown at the top of FIGURE 3) and a diaphragm thickness waveform (shown at the bottom of FIGURE 3) are shown. A dashed line indicates the onset of inspiratory effort. The onset of diaphragm thickening starts during the expiration phase since the intrinsic PEEP needs to be overcome first. In additional embodiments also the pressure waveforms can be displayed.

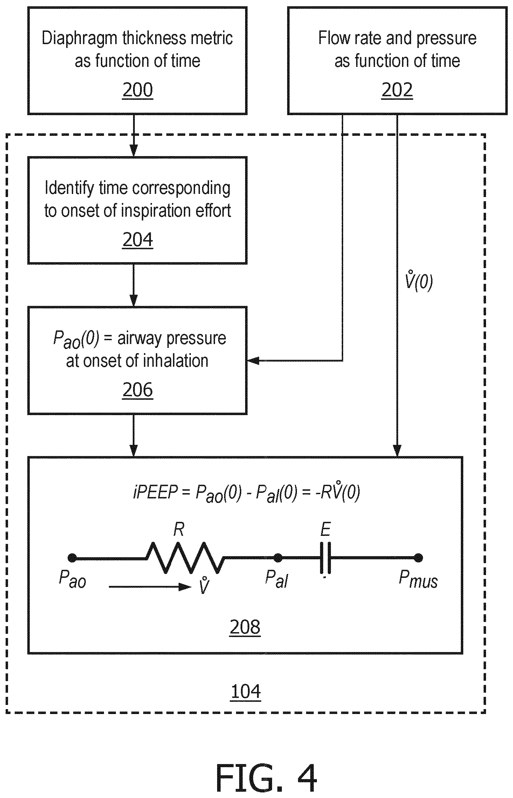

With reference to FIGURE 4, an example of one implementation of the iPEEP calculation **104** of FIGURE 2 is shown by way of a flowchart. The data input are the diaphragm thickness metric as a function of time **200** determined as described above, and the respiratory data comprising the flow rate V and airway pressure P as a function of time **202** both of which are routinely measured during mechanical ventilation. In an operation **204,** a time $t = 0$ corresponding to the onset of inspiration effort is detected, for example by identifying the inflection point in the diaphragm thickness metric as a function of time **200** indicated by the vertical dashed lines in FIGURE 3. In an operation **206,** the airway pressure $P_{ao}(0)$ at the time $t = 0$ corresponding to onset of inspiration effort is obtained from the respiratory data **202.**

Next, in an operation **208** the iPEEP value is determined. iPEEP is the pressure difference between the airway ($P_{ao}$) and the alveoli ($P_{al}$) just before start of inhalation ($t = 0$). From a single compartment model indicated in the block **208** of FIGURE 4, it can be derived that this equals the product of respiratory resistance R and flow (V) according to Equation 1:

$$iPEEP = P_{ao}(0) - P_{al}(0) = -R\dot{V}(0)7 \qquad (1)$$

Respiratory resistance values can be determined as disclosed in, for example, U.S. Provisional App. No. 63/407,772, filed September 19, 2022

Once the iPEEP value is known, it can be suggested to increase the PEEP setting of a quantity equal to the iPEEP.. This "total" PEEP can be suggested as a new setpoint for a new PEEP value to eliminate iPEEP (and hence enabling the lung to completely empty because of the larger driving pressure). Also, from this iPEEP value, an exhalation time could be estimated and proposed for complete emptying of the lung. Other proposed actions may include reduction of the tidal volume and breathing rate. It should be noted that instead of suggesting above actions to the caregiver, these actions can also be automatically implemented and controlled in a closed-loop system. The onset of the respiratory muscle to determine the iPEEP could be obtained also with a surface EMG synchronized with the ventilator

Referring back to FIGURE 2, at an operation **105,** a representation **30** of the calculated PEEP value is displayed on the display device **14.** In some examples, when multiple ultrasound patches **20** are attached to different locations on the chest of the patient **P,** the representation **30** can include a color map showing distribution of activity of the diaphragm.

In some embodiments, at an operation **106,** the mechanical ventilator **2** can be controlled to adjust one or more parameters of the mechanical ventilation therapy delivered to the patient based on the calculated diaphragm thickness metric. For example, an adjustment of at least one mechanical ventilation setting of the mechanical ventilator **2** can be determined based on the calculated PEEP value. The determined adjustment can be applied to the mechanical ventilator **2** to adjust the mechanical ventilation therapy to the patient **P.** The determined adjustment can be displayed on the display device **14** as a proposed adjustment to adjust the mechanical ventilation therapy to the patient **P.** The determined adjustment can be, for example, a change in an exhalation time, a change in a pressure applied by the mechanical ventilator **2** during exhalation, and so forth.

In some embodiments, instead of calculating the diaphragm thickness metric at the operation **103,** a negative pressure value can be determined based on respiratory effort by the patient **P.** In response to the calculated iPEEP value being above a threshold, a modified configuration of the mechanical ventilator **2** effective to apply a negative pressure to the patient during exhalation(or inferior to the PEEP settings) by the patient **P** during the mechanical ventilation therapy can be determined to assist with exhalation by the patient **P.** The determined modified configuration can be displayed on the display device **14,** or the mechanical ventilator **2** can be controlled to implement the determined modified configuration.

In this embodiment, the negative pressure is applied on an exhalation limb **7** of the patient breathing circuit **5** (see FIGURE 1) during the exhalation phase to help the exhalation by generating a larger pressure differential between the alveoli and the airways (i.e. to enable a faster exhalation). Viewed another way, the negative pressure applied to the exhalation limb 7 operates to actively draw air out of the lungs of the patient **P,** thus removing the residual air from the lungs that otherwise results in the undesirable positive value of iPEEP. The value of the negative pressure could be calculated from the intrinsic PEEP value as determined using a model. The negative pressure value could be suggested to the caregiver or automatically implemented and controlled in a closed-loop system. In cases of expiratory flow limitation, a reduction of the airway pressure during the exhalation phase may not result in a reduction of the flow before start of a new inhalation. However, monitoring the changes in the flow just before start of a new inhalation upon applied changes in airway pressure during the exhalation phase allows to detect presence of expiratory flow limitation. This could be indicated to the caregiver. If expiratory flow limitation is detected, a higher PEEP value can be suggested or automatically implemented using a closed-loop solution.

In some embodiments, instead of calculating the diaphragm thickness metric at the operation 103, a diaphragmatic excursion value of the diaphragm can instead be determined for successive breaths based on the ultrasound imaging data **24** as a function of time. An alert **30** can be displayed on the display device **14** in response to an increase or decrease of the diaphragmatic excursion values for the successive breaths over time satisfying an alert criterion.

In this embodiment, with ultrasound, it is possible to measure the excursion of the diaphragm. Provided the excursion can be measured in an absolute manner, a gradual shift would indicate an increasing expansion of the lungs due to dynamic hyperinflation. This provides an additional (i.e., indirect) manner to detect intrinsic PEEP with ultrasound. The advantage is that it allows to directly measure the cumulative effect of the intrinsic PEEP, instead of integrating a flow curve. This information is meaningful because the expansion of the chest activates receptors which causes arousals and discomfort. Additionally, an expansion of the lungs increases the risk of overstretching and VILI.

In some embodiments, the PEEP calculation operation **104** can include determining lung sliding of lungs of the patient for successive breaths based on speckle tracking performed on the ultrasound imaging data **24** as a function of time. An alert 30 can be displayed on the display device **14** in response to an increase or decrease of the lung sliding for the successive breaths over time satisfying an alert criterion.

In this embodiment, while the excursion of the diaphragm provides direct information on the lung volume and thus on a progressively increasing end-expiratory volume due to intrinsic PEEP, indirect information is also available from the pleura. Lung Ultrasound with speckle tracking allows quantifying lung sliding. It is expected that with progressively increasing lung volume the speckle position in the end-expiratory breathing phase is moving caudally while at the same time the distance between speckles is increasing. This effect can be small from breath to breath, but it could be detected over several breathing cycles and could thus trigger a warning. This embodiment would measure the cumulative effect of intrinsic PEEP.

In some embodiments, the electronic controller **13** is programmed to control the mechanical ventilator **2** having an inhalation limb and an exhalation limb to perform mechanical ventilation of a patient; and during the mechanical ventilation, in response to determining an iPEEP is higher than a threshold adjust the mechanical ventilation of the patient to apply a negative pressure on the exhalation limb during an exhalation phase of the mechanical ventilation of the patient.

In some embodiments, the electronic controller **13** is programmed to determine a diaphragmatic excursion value of a diaphragm of a patient undergoing mechanical ventilation for successive breaths based on ultrasound imaging data of the diaphragm as a function of time; and display, on the display device **14,** an alert in response to an increase or decrease of the diaphragmatic excursion values for the successive breaths over time satisfying an alert criterion.

In some embodiments, the electronic controller **13** is programmed to determine lung sliding of lungs of a patient undergoing mechanical ventilation for successive breaths based on ultrasound imaging data of the lungs of the patient as a function of time; and display, on the display device **14,** an alert in response to an increase or decrease of the lung sliding for the successive breaths over time satisfying an alert criterion.

The disclosure has been described with reference to the preferred embodiments. Modifications and alterations may occur to others upon reading and understanding the preceding detailed description. It is intended that the exemplary embodiment be construed as including all such modifications and alterations insofar as they come within the scope of the appended claims

**Claims**

1.  A respiration monitoring device, comprising at least one electronic processor programmed to perform a respiration monitoring method including:

    receiving ultrasound imaging data of a diaphragm of a patient as a function of time during inspiration and expiration while the patient undergoes mechanical ventilation therapy with a mechanical ventilator;
    receiving respiratory data of the patient as a function of time during the inspiration and expiration while the patient undergoes the mechanical ventilation therapy;
    calculating an intrinsic positive end-expiratory pressure (iPEEP) value for the patient based on the ultrasound imaging data and the respiratory data;
    displaying, on a display device, a representation of the calculated iPEEP value

    the respiration monitoring device comprising:

    an ultrasound imaging device including an ultrasound patch attached to a portion of the patient, wherein the at least one electronic processor controls the ultrasound imaging device to receive the ultrasound imaging data of the diaphragm of the patient from the ultrasound patch; and
    the mechanical ventilator configured to deliver mechanical ventilation therapy to the patient.

2.  The device of claim 1, wherein the method further includes:

    determining a diaphragm thickness metric as a function of time based on the received ultrasound imaging data of the diaphragm of the patient;
    wherein the iPEEP value is calculated based on the diaphragm thickness metric as a function of time and the respiratory data.

3.  The device of claim 2, wherein the calculating of the iPEEP value includes:

    identifying a time corresponding to start of inhalation based on the determined diaphragm thickness metric; and
    calculating the iPEEP value at the identified time corresponding to start of inhalation.

4.  The device of claim 2, wherein the calculating of the iPEEP value includes:

    identifying a time corresponding to start of inhalation based on the received respiratory data; and
    calculating the iPEEP value at the identified time corresponding to start of inhalation.

5.  The device of claim 4, wherein the iPEEP value is calculated as iPEEP = -RV(t) where R is a respiratory resistance of lungs of the patient and V(t) is an airflow at the identified time corresponding to start of inhalation which is part of the received respiratory data of the patient.

6.  The device of claim 1, wherein the received respiratory data of the patient as a function of time includes a ventilator flow waveform generated during the mechanical ventilation therapy, and calculating the iPEEP value comprises:

    detecting an onset of inspiratory effort by the patient based on the ultrasound imaging data;
    determining an air flow value from the lungs of the patient at the detected onset of inspiratory effort by the patient from the ventilator flow waveform; and
    calculating the iPEEP value based on the determined air flow value from the lungs of the patient at the detected onset of inspiratory effort by the patient.

7.  The device of claim 6, wherein the method further includes:
    triggering the mechanical ventilator to initiate a breath based on the determined onset of inspiratory effort by the patient.

8.  The device of claim 1, wherein the method further includes:

    determining a diaphragmatic excursion value of the diaphragm for successive breaths based on the ultrasound imaging data as a function of time; and

displaying, on the display device, an alert in response to an increase or decrease of the diaphragmatic excursion values for the successive breaths over time satisfying an alert criterion.

9. The device of claim 2, wherein calculating the iPEEP value from the calculated diaphragm thickness metric comprises:

determining lung sliding of lungs of the patient for successive breaths based on speckle tracking performed on the ultrasound imaging data as a function of time; and
displaying, on the display device, an alert in response to an increase or decrease of the lung sliding for the successive breaths over time satisfying an alert criterion.

10. The device of claim 1, wherein the method further includes:

determining a negative pressure value based on a respiratory effort of the patient; and
in response to the calculated iPEEP value being above a threshold, determining a modified configuration of the mechanical ventilator effective to apply a negative pressure to the patient during exhalation by the patient during the mechanical ventilation therapy to assist with exhalation by the patient; and
one of (i) displaying, on the display device, the determined modified configuration, or (ii) controlling the mechanical ventilator to implement the determined modified configuration.

11. The device of claim 1, wherein the method further includes:

determining an updated iPEEP value applied by the mechanical ventilator from the received respiratory data;
calculating a total PEEP value as a sum of the calculated iPEEP value and the determined updated iPEEP value.

12. The device of claim 1, wherein the method further includes:

determining an adjustment of at least one mechanical ventilation setting of the mechanical ventilator based on the calculated iPEEP value; and
one of: (i) applying the determined adjustment to the mechanical ventilator or (ii) displaying, on the display device, the determined adjustment as a proposed adjustment.

13. The device of claim 12, wherein the determined adjustment includes one of:

a change in an exhalation time; and
a change in a pressure applied by the mechanical ventilator during exhalation.

**Patentansprüche**

1. Vorrichtung zur Überwachung der Atmung, umfassend mindestens einen elektronischen Prozessor, der so programmiert ist, dass er ein Verfahren zur Überwachung der Atmung durchführt, das Folgendes einschließt:

Empfangen von Ultraschallbildgebungsdaten des Zwerchfells eines Patienten als Funktion der Zeit, während der Inspiration und der Exspiration, während sich der Patient einer mechanischen Beatmungstherapie mittels einer mechanischen Beatmungsvorrichtung unterzieht;
Empfangen von Atmungsdaten des Patienten als Funktion der Zeit, während der Inspiration und der Exspiration, während sich der Patient der mechanischen Beatmungstherapie unterzieht;
Berechnen des Wertes eines intrinsischen positiven endexspiratorischen Drucks (iPEEP) für den Patienten basierend auf den Ultraschallbildgebungsdaten und den Atmungsdaten;
Anzeigen, auf einer Anzeigevorrichtung, einer Darstellung des berechneten iPEEP-Wertes, wobei die Vorrichtung zur Überwachung der Atmung umfasst:

eine Ultraschallbildgebungsvorrichtung, die ein Ultraschallpflaster einschließt, das an einem Abschnitt des Patienten angebracht ist, wobei der mindestens eine elektronische Prozessor die Ultraschallbildgebungsvorrichtung steuert, um die Ultraschallbildgebungsdaten des Zwerchfells des Patienten vom Ultraschallpflaster zu empfangen; und

die mechanische Beatmungsvorrichtung dafür konfiguriert ist, dem Patienten eine mechanische Beatmungstherapie bereitzustellen.

2. Vorrichtung nach Anspruch 1, wobei das Verfahren ferner Folgendes einschließt:

Bestimmen eines Zwerchfelldickenmesswerts als Funktion der Zeit basierend auf den empfangenen Ultraschallbildgebungsdaten des Zwerchfells des Patienten;
wobei der iPEEP-Wert basierend auf dem Zwerchfelldickenmesswert als Funktion der Zeit und der Atmungsdaten berechnet wird.

3. Vorrichtung nach Anspruch 2, wobei das Berechnen des iPEEP-Wertes Folgendes einschließt:

Identifizieren eines Zeitpunkts, der dem Beginn der Inspiration entspricht, basierend auf dem bestimmten Zwerchfelldickenmesswert; und
Berechnen des iPEEP-Wertes zum identifizierten Zeitpunkt, der dem Beginn der Inspiration entspricht.

4. Vorrichtung nach Anspruch 2, wobei das Berechnen des iPEEP-Wertes Folgendes einschließt:

Identifizieren des Zeitpunkts, der dem Beginn der Inspiration entspricht, basierend auf den empfangenen Atmungsdaten; und
Berechnen des iPEEP-Wertes zum identifizierten Zeitpunkt, der dem Beginn der Inspiration entspricht.

5. Vorrichtung nach Anspruch 4,
wobei der iPEEP-Wert als iPEEP = -RV(t) berechnet wird, wobei R ein Atmungswiderstand der Lungen des Patienten ist und V(t) ein Luftstrom zum identifizierten Zeitpunkt ist, der dem Beginn der Inspiration entspricht und Teil der empfangenen Atmungsdaten des Patienten ist.

6. Vorrichtung nach Anspruch 1, wobei die empfangenen Atmungsdaten des Patienten als Funktion der Zeit eine während der mechanischen Beatmungstherapie erzeugte Beatmungsstromkurve einschließen und Berechnen des iPEEP-Wertes Folgendes umfasst:

Erfassen des Anfangs der Einatmungsbemühungen des Patienten basierend auf den Ultraschallbildgebungsdaten;
Bestimmen des Luftstromwerts aus den Lungen des Patienten beim erfassten Anfang der Einatmungsbemühungen des Patienten anhand der Beatmungsstromkurve; und
Berechnen des iPEEP-Wertes basierend auf dem bestimmten Luftstromwert aus den Lungen des Patienten beim erfassten Anfang der Einatmungsbemühungen des Patienten.

7. Vorrichtung nach Anspruch 6, wobei das Verfahren ferner Folgendes einschließt:
Auslösen der mechanischen Beatmungsvorrichtung zur Einleitung eines Atemzugs basierend auf dem bestimmten Anfang der Einatmungsbemühungen des Patienten.

8. Vorrichtung nach Anspruch 1, wobei das Verfahren ferner Folgendes einschließt:

Bestimmen eines Zwerchfellexkursionswertes des Zwerchfells für aufeinanderfolgende Atemzüge basierend auf den Ultraschallbildgebungsdaten als Funktion der Zeit; und
Anzeigen einer Warnung auf der Anzeigevorrichtung als Reaktion darauf, dass eine Erhöhung oder Verringerung der Zwerchfellexkursionswerte für die aufeinanderfolgenden Atemzüge im Laufe der Zeit ein Warnkriterium erfüllt.

9. Vorrichtung nach Anspruch 2, wobei das Berechnen des iPEEP-Wertes aus dem berechneten Zwerchfelldickenmesswert Folgendes umfasst:

Bestimmen der Lungengleitbewegung der Lungen des Patienten für aufeinanderfolgende Atemzüge basierend auf Speckle-Tracking, das auf den Ultraschallbildgebungsdaten als Funktion der Zeit durchgeführt wird; und
Anzeigen einer Warnung auf der Anzeigevorrichtung als Reaktion darauf, dass eine Erhöhung oder Verringerung der Lungengleitbewegung für die aufeinanderfolgenden Atemzüge im Laufe der Zeit ein Warnkriterium erfüllt.

**10.** Vorrichtung nach Anspruch 1, wobei das Verfahren ferner Folgendes einschließt:

Bestimmen eines Unterdruckwerts basierend auf den Atmungsbemühungen des Patienten; und
als Reaktion darauf, dass der berechnete iPEEP-Wert einen Schwellenwert überschreitet, Bestimmen einer modifizierten Konfiguration der Beatmungsvorrichtung, die wirksam ist, um während der Exspiration des Patienten während der mechanischen Beatmungstherapie einen Unterdruck anzuwenden, um die Exspiration des Patienten zu unterstützen; und
eindes von: (i) Anzeigen der bestimmten modifizierten Konfiguration auf der Anzeigevorrichtung oder (ii) Steuern der mechanischen Beatmungsvorrichtung zum Implementieren der bestimmten modifizierten Konfiguration.

**11.** Vorrichtung nach Anspruch 1, wobei das Verfahren ferner Folgendes einschließt:

Bestimmen eines aktualisierten iPEEP-Wertes, der von der mechanischen Beatmungsvorrichtung anhand der empfangenen Atmungsdaten angewendet wird;
Berechnen eines Gesamt-PEEP-Wertes als Summe des berechneten iPEEP-Wertes und des bestimmten aktualisierten iPEEP-Wertes.

**12.** Vorrichtung nach Anspruch 1, wobei das Verfahren ferner Folgendes einschließt:

Bestimmen einer Anpassung mindestens einer mechanischen Beatmungseinstellung der mechanischen Beatmungsvorrichtung basierend auf dem berechneten iPEEP-Wert; und
eines von: (i) Anwenden der bestimmten Einstellung an der mechanischen Beatmungsvorrichtung oder (ii) Anzeigen der bestimmten Anpassung als vorgeschlagene Anpassung auf der Anzeigevorrichtung.

**13.** Vorrichtung nach Anspruch 12, wobei die bestimmte Anpassung eine der folgenden einschließt:

eine Veränderung der Exspirationszeit; und
eine Änderung eines von der mechanischen Beatmungsvorrichtung während der Exspiration ausgeübten Drucks.

**Revendications**

**1.** Dispositif de surveillance respiratoire, comprenant au moins un processeur électronique programmé pour effectuer une procédé de surveillance respiratoire incluant :

la réception de données d'imagerie par ultrasons d'un diaphragme d'un patient en fonction du temps pendant l'inspiration et l'expiration alors que le patient subit une thérapie de ventilation mécanique avec un ventilateur mécanique ;
la réception de données respiratoires du patient en fonction du temps pendant l'inspiration et l'expiration alors que le patient subit la thérapie de ventilation mécanique ;
le calcul d'une valeur de pression expiratoire finale positive intrinsèque (iPEEP) pour le patient sur la base des données d'imagerie par ultrasons et des données respiratoires ;
l'affichage, sur un dispositif d'affichage, d'une représentation de la valeur iPEEP calculée, le dispositif de surveillance respiratoire comprenant :

un dispositif d'imagerie par ultrasons incluant un patch à ultrasons fixé sur une partie du patient, dans lequel l'au moins un processeur électronique commande le dispositif d'imagerie par ultrasons pour recevoir les données d'imagerie par ultrasons du diaphragme du patient à partir du patch à ultrasons ; et
le ventilateur mécanique configuré pour administrer une thérapie de ventilation mécanique au patient.

**2.** Dispositif selon la revendication 1, dans lequel le procédé inclut en outre :

la détermination d'une mesure de l'épaisseur du diaphragme en fonction du temps sur la base des données d'imagerie par ultrasons reçues du diaphragme du patient ;
dans lequel la valeur iPEEP est calculée sur la base de l'épaisseur du diaphragme en fonction du temps et des données respiratoires.

**3.** Dispositif selon la revendication 2, dans lequel le calcul de la valeur iPEEP inclut :

l'identification d'un moment correspondant au début de l'inspiration sur la base de la mesure de l'épaisseur du diaphragme déterminée ; et
le calcul de la valeur iPEEP au moment identifié correspondant au début de l'inspiration.

**4.** Dispositif selon la revendication 2, dans lequel le calcul de la valeur iPEEP inclut :

l'identification d'un moment correspondant au début de l'inspiration sur la base des données respiratoires reçues ; et
le calcul de la valeur iPEEP au moment identifié correspondant au début de l'inspiration.

**5.** Dispositif selon la revendication 4,
dans lequel la valeur iPEEP est calculée comme iPEEP = -RV(t) où R est une résistance respiratoire des poumons du patient et V(t) est un débit d'air au moment identifié correspondant au début de l'inspiration qui fait partie des données respiratoires reçues du patient.

**6.** Dispositif selon la revendication 1, dans lequel les données respiratoires reçues du patient en fonction du temps incluent une forme d'onde du débit du ventilateur générée pendant la thérapie de ventilation mécanique et le calcul de la valeur iPEEP comprend :

la détection d'un début d'un effort inspiratoire de la part du patient sur la base des données d'imagerie par ultrasons ;
la détermination d'une valeur du débit d'air des poumons du patient au début détecté de l'effort inspiratoire de la part du patient à partir de la forme d'onde du débit du ventilateur ; et
le calcul de la valeur iPEEP sur la base de la valeur du débit d'air déterminé à partir des poumons du patient au début détecté de l'effort inspiratoire de la part du patient.

**7.** Dispositif selon la revendication 6, dans lequel le procédé inclut en outre :
le déclenchement du ventilateur mécanique pour initier une respiration sur la base du début déterminé de l'effort inspiratoire de la part du patient.

**8.** Dispositif selon la revendication 1, dans lequel le procédé inclut en outre :

la détermination de la valeur d'une excursion diaphragmatique du diaphragme pour des respirations successives sur la base des données d'imagerie par ultrasons en fonction du temps ; et
l'affichage, sur le dispositif d'affichage, d'une alerte en réponse à une augmentation ou une diminution des valeurs d'excursion diaphragmatique pour les respirations successives au fil du temps satisfaisant un critère d'alerte.

**9.** Dispositif selon la revendication 2, dans lequel le calcul de la valeur iPEEP à partir de la mesure de l'épaisseur du diaphragme calculée comprend :

la détermination du glissement pulmonaire des poumons du patient lors de respirations successives sur la base du suivi des speckles effectué sur les données d'imagerie par ultrasons en fonction du temps ; et
l'affichage, sur le dispositif d'affichage, d'une alerte en réponse à une augmentation ou une diminution du glissement pulmonaire pour les respirations successives au fil du temps satisfaisant un critère d'alerte.

**10.** Dispositif selon la revendication 1, dans lequel le procédé inclut en outre :

la détermination d'une valeur de pression négative sur la base d'un effort respiratoire du patient ; et
en réponse au fait que la valeur iPEEP calculée dépasse un seuil, la détermination d'une configuration modifiée du ventilateur mécanique efficace pour appliquer une pression négative au patient pendant l'expiration par le patient pendant la thérapie de ventilation mécanique pour faciliter l'expiration par le patient ; et
l'une des options suivantes : (i) l'affichage, sur le dispositif d'affichage, de la configuration modifiée déterminée, ou (ii) la commande du ventilateur mécanique pour mettre en œuvre la configuration modifiée déterminée.

**11.** Dispositif selon la revendication 1, dans lequel le procédé inclut en outre :

la détermination d'une valeur iPEEP mise à jour appliquée par le ventilateur mécanique à partir des données respiratoires reçues ;

le calcul d'une valeur PEEP totale comme la somme de la valeur iPEEP calculée et de la valeur iPEEP mise à jour déterminée.

12. Dispositif selon la revendication 1, dans lequel le procédé inclut en outre :

la détermination d'un ajustement d'au moins un paramètre de ventilation mécanique du ventilateur mécanique sur la base de la valeur iPEEP calculée ; et

l'une des options suivantes : (i) l'application de l'ajustement déterminé au ventilateur mécanique ou (ii) l'affichage, sur le dispositif d'affichage, de l'ajustement déterminé comme un réglage proposé.

13. Dispositif selon la revendication 12, dans lequel l'ajustement déterminé inclut l'une des options suivantes :

une modification du temps d'expiration ; et
une modification de la pression appliquée par le ventilateur mécanique pendant l'expiration.

1

Respiration
monitoring
method or process
100

15

18

24

14

30

2

13

5

6

8

7

P

20

16

FIG. 1

100

Acquire imaging data
101

Acquire respiratory data
102

Calculate diaphragm thickness metric
103

Calculate iPEEP
104

Display representation of iPEEP
105

Control ventilator based on iPEEP
106

FIG. 2

Flow
inflection
point

Diaphragm
thickness

FIG. 3

FIG. 4

**REFERENCES CITED IN THE DESCRIPTION**

**Patent documents cited in the description**

- US 63426069

- US 63407772

**Non-patent literature cited in the description**

- **EMANUELE BERNARDI**. *American Journal of Respiratory and Critical Care Medicine*, 01 August 2018, vol. 198 (3), ISSN 1073-449X, 392-396